# FASCICULE DE BREVET EUROPEEN

(11) **EP 0 979 816 B1**
(45) Date de publication et mention de la délivrance du brevet: **02.05.2003**
(21) Numéro de dépôt: 99402016.2
(22) Date de dépôt: 09.08.1999
(51) Int. Cl.: C07C 323/60, A61K 31/18

(54) **Nouveaux dérivés d'acide hydroxamique, leur procédé de préparation et les compositions pharmaceutiques qui les contiennent**
Hydroxamsäure-Derivate, Verfahren zu ihrer Herstellung und diese enthaltende pharmazeutische Zusammenstellungen
Hydroxamic acid derivatives, process for their preparation and pharmaceutical compositions containing them

(30) Priorité: 10.08.1998 FR 9810237
(43) Date de publication de la demande: 16.02.2000
(73) Titulaire: LES LABORATOIRES SERVIER, 92200 Neuilly sur Seine (FR)
(72) Inventeur: Hanessian, Stephen, Beaconsfield P.Q. H9W 5H3 (CA); Atassi, Ghanem, 92210 Saint Cloud (FR); Tucker, Gordon, 75017 Paris (FR); Caignard, Daniel-Henri, 78230 Le Pecq (FR); Renard, Pierre, 78150 Le Chesnay (FR)

(56) Documents cités:
- EP-A- 0 606 046
- WO-A-96/00214
- WO-A-96/40101
- L.J. MACPHERSON, ET AL.: "Discovery of CGS 27023A, a non-peptidic, potent, and orally active stromelysin inhibitor that blocks cartilage degradation in rabbits" JOURNAL OF MEDICINAL CHEMISTRY, vol. 40, no. 16, 1 août 1997 (1997-08-01), pages 2525-2532, XP002099324 Washington, DC, US
- YOSHINORI TAMURA: "Highly selective and orally active inhibitors of Type IV Collagenase (MMP-9 aand MMP-2): N-Sulfonylamino Acid Deivatives" JOURNAL OF MEDICINAL CHEMISTRY, vol. 41, 1998, pages 640-649, XP002072052 Washington, DC, US

## Description

La présente invention concerne de nouveaux dérivés d'acide hydroxamique, leur procédé de préparation et les compositions pharmaceutiques qui les contiennent. Ces nouveaux composés constituent de nouveaux inhibiteurs de métalloprotéases.

Le remodelage de la matrice extracellulaire est impliqué dans de nombreux processus physiologiques et pathologiques: développement embryonnaire, cicatrisation, angiogenèse normale et pathologique, dégénérescence des tissus conjonctifs et articulaires, cancers invasifs et métastatiques, par exemple.

Les métalloprotéinases de la matrice extracellulaire ('matrix metalloproteinases' ou MMPs), des enzymes décrites initialement comme impliquées dans la régulation de la formation et de la destruction du matériel extracellulaire, sont surexprimées au cours de tels événements et associées à la progression pathologique. Cette famille de protéases à zinc comporte au moins quatorze membres. Les principaux membres dont on connaît les substrats sont: les collagénases (MMP-1 interstitielle, MMP-8 neutrophilique et la collagénase-3 ou MMP-13), les gélatinases (collagénases de type IV MMP-2 et MMP-9, ou gélatinases A et B), la métalloélastase MMP-12, les stromélysines (dont la stromélysine-1 ou MMP-3) et les activateurs de la gélatinase A (MT-MMPs, seules MMPs possédant un domaine transmembranaire).

Dans la pathologie tumorale, ces enzymes, sécrétées par les cellules cancéreuses et les cellules normales du stroma péritumoral, participent directement à l'ouverture de voies de migration dans le tissu interstitiel pour les cellules endothéliales et tumorales, et indirectement au relargage et à la maturation de facteurs membranaires ou séquestrés dans la matrice extracellulaire (facteurs angiogéniques ou de croissance, médiateurs de l'inflammation comme le 'tumour necrosis factor'-alpha ou TNF-alpha,...), contribuant ainsi à toutes les étapes de la progression tumorale (croissance de la tumeur primaire, angiogenèse, invasion locale et établissement de métastases).
Des inhibiteurs de MMPs seraient donc particulièrement efficaces comme nouvelles entités pharmacologiques susceptibles d'enrayer la progression de nombreuses pathologies, dont le cancer.

Différents inhibiteurs de métalloprotéases ont été décrits dans la littérature. C'est le cas plus particulièrement des composés décrits dans les demandes de brevet EP 606 046, WO 96/40101, WO 96/00214 ou WO 97/27174 ainsi que dans l'article J. Med. Chem. 1998, 41, 640-649.

Les composés de la présente invention, outre le fait qu'ils soient nouveaux, se sont révélés être des inhibiteurs de métalloprotéases plus puissants que ceux décrits dans la littérature, ce qui les rend donc potentiellement utiles pour le traitement des cancers, des maladies rhumatismales comme l'arthrose et l'arthrite rhumatoïde, etc...

Plus spécifiquement, la présente invention concerne les composés de formule (I) : 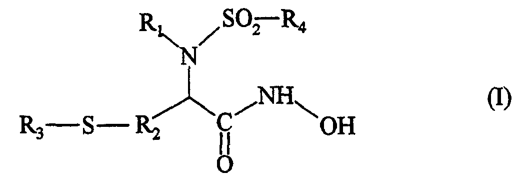 dans laquelle :
R₁ représente :
   - un groupement alkyle C₁-C₆ linéaire ou ramifié (éventuellement substitué par un ou plusieurs groupements, identiques ou différents indépendamment l'un de l'autre, choisis parmi hydroxy, halogène, alkoxy C₁-C₆ linéaire ou ramifié, mercapto, alkylthio C₁-C₆ linéaire ou ramifié, aryle, acyle C₁-C₆ linéaire ou ramifié, ou amino lui-même éventuellement substitué par un ou deux groupements, identiques ou différents, alkyle C₁-C₆ linéaire ou ramifié, cycloalkyle ou aryle),
   - un groupement acyle C₁-C₆ linéaire ou ramifié,
   - un groupement cycloalkyle,
   - un groupement aryle,
   - un hétérocycle,
   - un groupement aminocarbonylalkyle C₁-C₄ la partie amino étant éventuellement substituée par un groupement alkyle C₁-C₆ linéaire ou ramifié, ledit groupement alkyle C₁-C₆ étant éventuellement substitué par un ou plusieurs groupements, identiques ou différents, choisis parmi aryle, arylalkyle C₁-C₆ linéaire ou ramifié, cycloalkyle et alkylaminocarbonyle C₁-C₆ linéaire ou ramifié,
R₂ représente un groupement alkyléne C₁-C₄ linéaire ou ramifié,
R₃ représente un groupement X ou Y avec :
   - X représente un groupement alkyle C₁-C₆ linéaire ou ramifié, acyle C₁-C₆ linéaire ou ramifié, alkoxycarbonyle C₁-C₆ linéaire ou ramifié, aminoalkyle C₁-C₆ linéaire ou ramifié (la partie amino étant elle-même éventuellement substituée par un ou deux groupements, identiques ou différents, alkyle C₁-C₆ linéaire ou ramifié), hydroxyalkyle C₁-C₆ linéaire ou ramifié, carboxyalkyle C₁-C₆ linéaire ou ramifié, aminocarbonylalkyle C₁-C₆ linéaire ou ramifié, mercaptoalkyle C₁-C₆ linéaire ou ramifié, cycloalkyle, aryle ou un hétérocycle,
   - Y représente un groupement de formule T-U-V- (la partie V étant reliée à l'atome de soufre) dans laquelle :
      - T représente un groupement aryle ou un hétérocycle,
      - U représente une liaison simple, un atome de soufre, d'oxygène, un groupement NH ou C=O, ou un groupement de formules -R₈O-, -R₈S-, -R₈NH-, -R₈OR₉--R₈SR₉-, -R₈-NH-R₉-, -R₈-CO-R₉-, -R₉-, dans lesquelles R₈ représente un groupement alkylène C₁-C₆ linéaire ou ramifié, et R₉ représente un groupement arylène ou hétéroaryléne, étant entendu que dans cesdits groupements, R₈ est relié à la partie T du groupement Y et R₉ ou l'hétéroatome est relié à la partie V du groupement Y,
      - V représente un groupement alkylène C₁-C₆ linéaire ou ramifié,
R₄ représente :
   - soit, quand R₃ représente un groupement Y, un groupement alkyle C₁-C₆ linéaire ou ramifié, cycloalkyle, aryle, arylalkyle C₁-C₆ linéaire ou ramifié, cycloalkylalkyle C₁-C₆ linéaire ou ramifié, alkyle C₁-C₆ linéaire ou ramifié substitué par un hétérocycle, ou un hétérocycle,
   - soit, quand R₃ représente un groupement X ou Y, un groupement biaryle, arylhétéroaryle, ou hétéroarylaryle,
leurs isomères ainsi que leurs sels d'addition à un acide ou à une base pharmaceutiquement acceptable.

Etant entendu que :
- par groupement cycloalkyle, on comprend un système mono ou bicyclique comportant de 3 à 10 atomes de carbone,
- par groupement aryle, on comprend un groupement phényle, naphtyle, tétrahydronaphtyle, dihydronaphtyle,indène ou dihydroindène, chacun de ces groupements étant éventuellement substitué de façon identique ou différente, par un ou plusieurs atomes d'halogène, groupements hydroxy, cyano, nitro, alkyle C₁-C₆ linéaire ou ramifié, trihalogénoalkyle C₁-C₆ linéaire ou ramifié, alkoxy C₁-C₆ linéaire ou ramifié, acyle C₁-C₆ linéaire ou ramifié, carboxy, alkoxycarbonyle C₁-C₆ linéaire ou ramifié, amino lui-même étant éventuellement substitué par un ou deux groupements, identiques ou différents, alkyle C₁-C₆ linéaire ou ramifié,
- par groupement biaryle on comprend un groupement aryle dont l'un des carbones du cycle est substitué par un second groupement aryle,
- par hétérocycle, on entend un groupement mono ou bicyclique, saturé ou insaturé, de 4 à 12 chaînons, contenant un, deux ou trois hétéroatomes, identiques ou différents, choisis parmi oxygène, azote ou soufre, étant entendu que l'hétérocycle peut être éventuellement substitué, de façon identique ou différente, par un ou plusieurs atomes d'halogène, groupements hydroxy, alkyle C₁-C₆ linéaire ou ramifié, trihalogénoalkyle C₁-C₆ linéaire ou ramifié, alkoxy C₁-C₆ linéaire ou ramifié, amino substitué éventuellement par un ou plusieurs groupements alkyle C₁-C₆ linéaire ou ramifié,
- par hétéroaryle, on comprend un hétérocycle insaturé à caractère aromatique.

D'une façon avantageuse, les composés préférés de l'invention sont ceux pour lesquels R₁ représente un groupement alkyle C₁-C₆ linéaire ou ramifié et plus particulièrement R₁ représente un groupement isobutyle.

Selon une variante avantageuse de l'invention, les composés préférés sont ceux pour lesquels R₃ représente un groupement X tel que défini précédemment et R₄ représente un groupement biaryle, arylhétéroaryle ou hétéroarylaryle.

Selon une autre variante avantageuse de l'invention, les composés préférés sont ceux pour lesquels R₃ représente un groupement Y tel que défini précédemment et R₄ représente un groupement alkyle C₁-C₆ linéaire ou ramifié, cycloalkyle, aryle, arylalkyle C₁-C₆ linéaire ou ramifié, cycloalkylalkyle C₁-C₆ linéaire ou ramifié, hétérocycloalkyléne C₁-C₆ linéaire ou ramifié ou un hétérocycle.

Selon une troisième variante particulièrement avantageuse, les composés préférés de l'invention sont ceux pour lesquels R₃ représente un groupement Y tel que défini précédemment et R₄ représente un groupement biaryle, arylhétéroaryle ou hétéroarylaryle.

Les composés préférés de l'invention sont les composés de formule (I) qui sont :
- l'acide 4-(benzylsulfanyl)-2-{isobutyl[(4-méthoxyphényl)sulfonyl]amino}butanhydroxamique,
- l'acide 4-{[4-(phényl)benzyl]sulfanyl}-2-{isobutyl-[(4-méthoxyphényl)sulfonyl]amino} butanhydroxamique,
- l'acide 4-(benzylsulfanyle)-2-{isobutyl-[(4-biphényl)sulfonyl]amino}butanhydroxamique,
- l'acide 4-{[4-(benzyloxy)benzyl]sulfanyl}-2-{isobutyl-[(4-méthoxyphényl)sulfonyl]amino}-butanhydroxamique,
- l'acide 2-{isobutyl-[(4-biphényl)sulfonyl]amino}-4-(méthylsulfanyl)butanhydroxamique,
- et l'acide 2-{[2-(benzhydrylamino)-2-oxoéthyl]-[(4-méthoxyphényl)sulfonyl]amino}-4-(benzylsulfanyl)butanhydroxamique.

Les isomères ainsi que les sels d'addition à un acide ou à une base pharmaceutiquement acceptable, des composés préférés font partie intégrante de l'invention.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

L'invention s'étend également au procédé de préparation des composés de formule (I), caractérisé en ce qu'on utilise comme produit de départ, un composé de formule (II) : 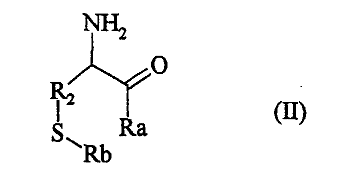 dans laquelle R₂ est tel que défini dans la formule (I) et, soit Ra et Rb forment une liaison simple reliant le groupement carbonyle à l'atome de soufre, soit Ra représente un groupement alkoxy C₁-C₆ linéaire ou ramifié, et Rb représente un groupement alkyle C₁-C₆ linéaire ou ramifié,
composés de formule (II), dont on substitue, selon des conditions classiques de synthèse organique, la fonction amine primaire, par un composé de formule (II') :

R₁ - Z (II')

dans laquelle R₁ est tel que défini dans la formule (I) et Z représente un groupement libérable usuel de la chimie organique,
pour conduire aux composés de formule (III), 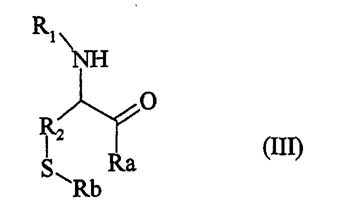 dans laquelle R₁ et R₂ ont la même signification que dans la formule (I) et, Ra et Rb sont tels que définis précédemment,
composés de formule (III), que l'on traite en condition basique, avec un composé de formule (IV) :

R₄ - SO₂ - Cl (IV)

dans laquelle R₄ est tel que défini dans la formule (I),
pour conduire aux composés de formule (V), 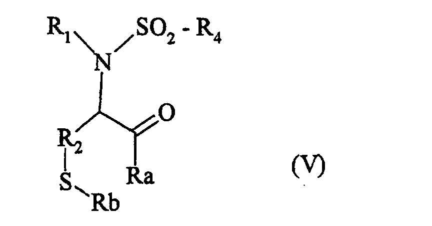 dans laquelle R₁, R₂, R₄, Ra et Rb sont tels que définis précédemment,
composés de formule (V) que l'on traite, dans le cas où Ra et Rb forment une liaison simple, en présence de méthanol et de sodium, par un composé de formule (VI) :

R₃ - Hal (VI)

dans laquelle R₃ est tel que défini dans la formule (I) et Hal représente un atome d'halogène, pour conduire aux composés de formule (VII) : 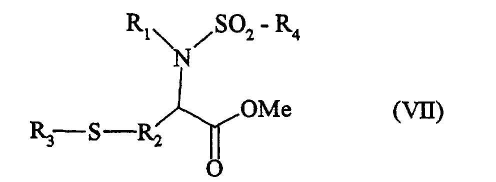 dans laquelle R₁, R₂, R₃ et R₄ sont tels que définis dans la formule (I), l'ensemble des composés de formule (VII) et (V) formant les composés de formule (IX) : 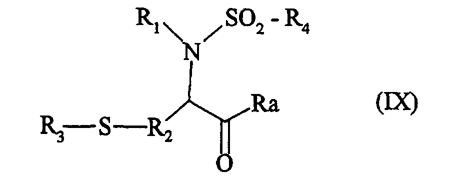 dans laquelle R₁, R₂, R₃, R₄ et Ra sont tels que définis précédemment,
composés de formule (IX) dont on hydrolyse la fonction ester, selon des conditions classiques, pour conduire aux composés de formule (X) : 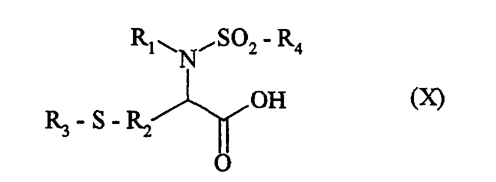 dans laquelle R₁, R₂, R₃ et R₄ sont tels que définis précédemment,
composés de formule (X) que l'on traite soit directement par de l'hydrochlorure d'hydroxylamine, soit par une hydroxylamine-O-substituée que l'on déprotège par la suite selon des conditions opératoires classiques, pour conduire aux composés de formule (I) tels que définis précédemment,
composé de formule (I) que l'on purifie, le cas échéant, selon une technique classique de purification, dont on sépare éventuellement les isomères selon une technique classique de séparation et que l'on transforme, si on le souhaite, en ses sels d'addition à un acide ou à une base pharmaceutiquement acceptable.

Les composés de formule (II), (II'), (IV) et (VI) sont soit des composés commerciaux, soit obtenus selon les méthodes classiques de synthèse organique.

La présente invention a également pour objet les compositions pharmaceutiques renfermant comme principe actif au moins un composé de formule (I), ses isomères optiques ou un de ses sels d'addition à une base ou un acide pharmaceutiquement acceptable, seul ou en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublinguaux, les gélules, les capsules, les suppositoires, les crèmes, les pommades, les gels dermiques, les préparations injectables ou buvables, les aérosols, les gouttes oculaires ou nasales, etc...

La posologie utile varie selon l'âge et le poids du patient, la voie d'administration, la nature et la sévérité de l'affection et la prise de traitements éventuels associés et s'échelonne de 1 à 500 mg en une ou plusieurs prises par jour.

Les exemples suivants illustrent l'invention mais ne la limitent en aucune façon.

Les produits de départ utilisés sont des produits connus ou préparés selon des modes opératoires connus.
Les différents stades de synthèse conduisent à des intermédiaires de synthèse, utiles pour la préparation des composés de l'invention.

Les structures des composés décrits dans les exemples et les stades de synthèse ont été déterminées selon les techniques spectrophotométriques usuelles (infrarouge, RMN, spectomètre de masse...).

### EXEMPLE 1 : Acide 4-(benzylsulfanyl)-2-{isobutyl[(4-méthoxyphényl)sulfonyl] amino}butanhydroxamique

### Stade A : 3 -(Isobutylamino)tétrahydro-2-thiophénone

A une solution de 4,23 mmol d'hydrochlorure de D-homocystéine thiolactone et 8,46 mmol d'isobutyraldéhyde dans 20 ml de méthanol sont ajoutées, à 0°C, sous atmosphère inerte, 4,6 mmol de triéthylamine. Après 4 heures d'agitation à température ambiante, la réaction est refroidie à 0°C et 8,8 mmol de NaCNBH₃ sont ajoutés lentement en 40 minutes. Après 30 minutes d'agitation à 0°C, la réaction est hydrolysée, puis extraite à l'éther. Les phases organiques rassemblées sont ensuite lavées par une solution saturée en NaCI, séchées sur sulfate de sodium, filtrées et concentrées sous pression réduite. Une chromatographie sur gel de silice (dichlorométhane/méthanol : 99/1) permet d'isoler le produit attendu sous forme de sirop.

### StadeB : N1-Isobutyl-N1-(2-oxotétrahydro-3-thiophényl)-4-méthoxy-1-benzènesulfonamide

A une solution de 2,5 mmol du composé obtenu dans le stade A dans 15 ml de dichlorométhane sont additionnées, à 0°C, 5 mmol de N-méthylmorpholine et 2,5 mmol de chlorure de 4-méthoxyphénylsulfonyle. Après 12 heures d'agitation à température ambiante, le milieu réactionnel est jeté dans de l'eau puis extrait au dichlorométhane. Les phases organiques rassemblées sont lavées par une solution d'acide chlorhydrique 2N, puis par une solution à 5 % de NaHCO₃, puis par de l'eau. Après séchage sur sulfate de sodium, filtration et évaporation, une chromatographie sur gel de silice (dichlorométhane/méthanol : 20/1) permet d'isoler le produit attendu.
**Point de fusion : 92°C**

### Stade C : 4-(Benzylsulfanyl)-2-{isobutyl[(4-méthoxyphényl)sulfonyl]amino}butanoate de méthyle

A une solution de 1,26 mmol de sodium dans 3 ml de méthanol sont additionnées, à température ambiante, 1,1 mmol du composé obtenu dans le stade B. Après 15 minutes d'agitation, 1,1 mmol de bromure de benzyle sont additionnées et l'agitation est maintenue pendant deux heures. Après évaporation du méthanol, on obtient un solide qui est trituré dans l'acétate d'éthyle et filtré. Une évaporation du filtrat permet d'obtenir un résidu qui est purifié par chromatographie sur gel de silice (dichlorométhane/méthanol : 20/1) permettant d'isoler le produit attendu sous forme de sirop.
**Spectre de masse : FAB**^{**+**} **: [M**^{**+**} **+ 1] : m/z = 466**

### Stade D : Acide 4-(benzylsulfanyl)-2-{isobutyl[(4-méthoxyphényl)sulfonyl] amino}butanoïque

A 0,95 mmol du composé obtenu dans le stade C dans un mélange 3/1 : dioxane/eau sont additionnées 1,77 mmol de potasse. Après 3 heures d'agitation à 50°C, le dioxane est évaporé, la phase aqueuse résiduelle est diluée avec de l'eau, acidifiée jusqu'à pH = 2 par addition d'une solution d'acide chlorhydrique à 5 %, puis extraite à l'acétate d'éthyle. Les phases organiques rassemblées sont lavées, séchées, filtrées puis évaporées sous pression réduite. Une chromatographie sur gel de silice (dichlorométhane/méthanol : 93/7) permet d'isoler le produit attendu sous forme de sirop.

### Stade E : N-tert-Butoxy-4-(benzylsulfanyl)-2-{isobutyl[(4-méthoxyphényl) sulfonyl]amino}butanamide

0,6 mmol du composé obtenu dans le stade D, 0,6 mmol de 1-hydroxybenzotriazole, 3 mmol de N-méthylmorpholine et 1,2 mmol d'hydrochlorure de O-tert-butylhydroxylamine sont dissouts dans 9 ml de dichlorométhane. 0,78 mmol d'hydrochlorure de N-[(diméthylamino)propyl]-N-éthylcarbodiimide est ensuite additionnée à cette solution et la réaction est agitée à température ambiante pendant 12 heures. Le milieu réactionnel est alors dilué par addition d'eau, puis extrait au dichlorométhane. Les phases organiques rassemblées sont lavées par une solution saturée en NaCI, séchées sur sulfate de sodium, filtrées et concentrées sous pression réduite. Une chromatographie sur gel de silice (dichlorométhane/méthanol : 99/1) permet d'isoler le produit sous forme de sirop.
**Spectre de masse : FAB**^{**+**} **: (M**^{**+**} **+ 1) : m/z = 523**

### StadeF : Acide 4-(benzylsulfanyl)-2-{isobutyl[(4-méthoxyphényl) sulfonyl] amino} butanhydroxamique

Une solution contenant 0,31 mmol du composé obtenu dans le stade E dans 2 ml de dichlorométhane et 2 ml d'acide trifluoroacétique est agitée pendant 6 heures à température ambiante, puis concentrée sous pression réduite et chromatographiée sur gel de silice (dichlorométhane/méthanol : 98/2). On obtient un résidu sirupeux qui est alors dissout dans l'acétate d'éthyle. La solution est ensuite filtrée sur célite puis concentrée sous pression réduite, permettant d'isoler le produit attendu sous forme de sirop.
**Spectre de masse : FAB**^{**+**} **: (M**^{**+**} **+ 1) : m/z = 467 (M**^{**+**} **- CONHOH) : m/z = 406**

### EXEMPLE 2 : Acide 4-{[4-(phényl)benzyl]sulfanyl}-2-{isobutyl-[(4-méthoxyphényl) sulfonyl]amino}butanhydroxamique

Le produit est obtenu selon le procédé décrit dans l'exemple 1 en utilisant au stade C, comme réactif, le bromure de 4-(phényl)benzyle.

### Stade C : 4-{[4-(Phényl)benzyl]sulfanyl}-2-{isobutyl-[(4-méthoxyphényl)sulfonyl]amino} butanoate de méthyle

### Stade D : Acide 4-{[4-(phényl)benzyle]sulfanyl}-2-{isobutyl-[(4-méthoxyphényl)sulfonyl] amino}butanoïque

### Stade E : N-tert-Butoxy-4-{[4-(phényl)benzyl]sulfanyl}-2-{isobutyl-[(4-méthoxyphényl) sulfonyl]amino}butanamide

### Stade F : Acide 4-{[4-(phényl)benzyl]sulfanyl}-2-{isobutyl-[(4-méthoxyphényl)sulfonyl] amino}butanhydroxamique

### EXEMPLE 3 : Acide 4-(benzylsulfanyl)-2-{isobutyl-(4-biphényl)sulfonyl]amino} butanhydroxamique

Le produit est obtenu selon le procédé décrit dans l'exemple 1 en utilisant au stade B, comme réactif le chlorure de 4-biphénylsulfonyle.

### Stade B : N1-Isobutyl-N1-(2-oxotétrahydro-3-thiophényl)-4-phény(-1-benzènesulfonamide

### Stade C : 4-(Benzylsulfanyl)-2-{isobutyl-[(4-biphényl)sulfonyl]amino}butanoate de méthyle

### Stade D : Acide 4-[benzylsulfanyl)-2-{isobutyl-[(4-biphényl)sulfonyl]amino}butanoïque

### Stade E : N-tert-Butoxy-4-(benzylsulfanyl)-2-{isobutyl-[(4-biphényl)sulfonyl]amino} butanamide

### Stade F : Acide 4-(benzylsulfanyl)-2-{isobutyl-(4-biphényl)sulfonyl] amino}butanhydroxamique

### EXEMPLE 4 : Acide 4-{[4-(benzyloxy)benzyl]sulfanyl}-2-{isobutyl-[(4-méthoxyphényl)sulfonyl]amino}butanhydroxamique

Le produit est obtenu selon le procédé décrit dans l'exemple 1, des stades A à D, en utilisant au stade C comme réactif le bromure de 4-(benzyloxy)benzyle, puis en effectuant le stade G décrit ci-après.

### Stade C : 4-{[4-(Benzyloxy)benzyl]sulfanyl}-2-{isobutyl-[(4-méthoxyphényl)sulfonyl] amino}butanoate de méthyle

### Stade D : Acide 4-{[4-(benzyloxy)benzyl]sulfanyl}-2-{isobutyl-[(4-méthoxyphényl) sulfonyl]amino}butanoïque

### Stade G : Acide 4-{[4-(benzyloxy)benzyl]sulfanyl}-2-{isobutyl-[(4-méthoxyphényl) sulfonyl]amino}butanhydroxamique

1 mmol du composé obtenu dans le stade D, 1 mmol de 1-hydroxybenzotriazole, 5 mmol de N-méthylmorpholine et 2 mmol d'hydrochlorure d'hydroxylamine sont dissous dans 15 ml de dichlorométhane. 1,2 mmol d'hydrochlorure de N-[(diméthylamino)propyl]-N-éthylcarbodiimide sont ensuite additionnées à cette solution et la réaction est agitée à température ambiante pendant 12 heures. Le milieu réactionnel est ensuite hydrolysé par addition d'eau puis la phase aqueuse est extraite au dichlorométhane. Les phases organiques rassemblées sont lavées par une solution saturée en NaCl, séchées sur sulfate de sodium, filtrées et concentrées sous pression réduite. Une chromatographie sur gel de silice (dichlorométhane/méthanol : 98/2) permet d'isoler le produit attendu.

### EXEMPLE 5 : Acide 2-{isobutyl-[(4-biphényl)sulfonyl]amino}-4-(méthylsulfanyl) butanhydroxamique

### Stade H : Hydrochlorure de 2-amino-4-(méthylsulfanyl)butanoate de méthyle

A 15 ml de méthanol sont additionnés, à -10°C, 16,5 mmol de chlorure de thionyle puis, en petites fractions, 11 mmol de D-méthionine. Après addition, le milieu réactionnel est ramené à température ambiante. Après 12 heures, la réaction est concentrée sous pression réduite. On obtient des cristaux qui sont recristallisés dans un mélange éther/méthanol, permettant d'isoler le produit attendu.
**Point de fusion : 143°C**

### Stade I : 2-(Isobutylamino)-4-(méthylsulfanyl)butanoate de méthyle

A une solution de 3,9 mmol du composé obtenu dans le stade H et 7,8 mmol d'isobutyraldéhyde, dans 20 ml de méthanol sont ajoutés, à 0°C et sous atmosphère inerte, 4,3 mmol de triéthylamine. Après 6 heures de réaction à température ambiante, la réaction est refroidie à 0°C et 7,8 mmol de NaBH₄ sont additionnés pendant une période de 40 minutes. Après addition complète, l'agitation est maintenue 30 minutes à 0°C, puis la réaction est hydrolysée par addition d'une solution aqueuse de NaHCO₃. Après extraction à l'éther, les phases organiques rassemblées sont lavées par une solution saturée en NaCl, séchées sur sulfate de sodium, puis concentrées sous pression réduite. Une chromatographie sur gel de silice (dichlorométhane/méthanol : 99/1) permet d'isoler le produit attendu.

### Stade J : 2-{Isobutyl-[(4-phényl)phénylsulfonyl]amino}-4-(méthylsulfanyl)butanoate de méthyle

A une solution de 1,8 mmol du composé obtenu dans le stade 1 dans 15 ml de dichlorométhane sont additionnées, à 0°C, 3,71 mmol de triéthylamine et 1,8 mmol de chlorure de 4-biphénylsulfonyl. La réaction est ramenée à température ambiante, et agitée pendant 12 heures, puis jetée sur de l'eau et extraite au dichlorométhane. Les phases organiques rassemblées sont lavées par une solution d'acide chlorhydrique 2N, puis par une solution de NaHCO₃, séchées, filtrées et concentrées sous pression réduite. Une chromatographie sur gel de silice (dichlorométhane/méthanol : 20/1) permet d'isoler le produit attendu sous forme de sirop.

### Stade K : Acide 2-{isobutyl-[4-(biphényl)sulfonyl]amino}-4-(méthylsulfanyl) butanhydroxamique

Le produit obtenu dans le stade J est alors soumis à la procédure décrite dans l'exemple 1, du stade D au stade F, permettant d'obtenir le produit attendu.
**Spectre de masse : FAB**^{**+**} **: (M**^{**+**} **+ 1) : m/z = 437 ; [M**^{**+**} **- CONHOH] : m/z = 376**

### EXEMPLE 6 : Acide 2-{[2-(benzhydrylamino)-2-oxoéthyl][(4-méthoxyphényl) sulfonyl]amino]}-4-(benzylsulfanyl)butanhydroxamique

### Préparation 1 : tert-Butyl-2-[(2-oxotétrahydro-3-thiophényl)amino]acétate

A une solution de 5 mmol de thiolactone dans 10 ml d'acétonitrile sont additionnés à 0°C, 5,5 mmol de diisopropyléthylamine puis 5,5 mmol de tert-butylbromoacétate. Après 30 minutes d'agitation, la réaction est ramenée à température ambiante pendant 12 heures. Le milieu réactionnel est alors concentré sous pression réduite, puis chromatographié sur gel de silice (dichlorométhane/méthanol : 97/3) permettant d'isoler le produit attendu.

### Stade B : tert-Butyl-2-{[(4-méthoxyphényl)sulfonyl](2-oxotétrahydro-3-thiophényl) amino}acétate

On procède comme dans le stade B de l'exemple 1 en utilisant comme substrat le produit obtenu dans la préparation 1.
**Spectre de masse : FAB**^{**+**} **: [M**^{**+**} **+ 1] : m/z = 402**

### Stade C : 4-(Benzylsulfanyl)-2-{[2-(tert-butoxy)-2-oxoéthyl][(4-méthoxyphényl) sulfonyl] amino}butanoate de méthyle

On procède comme dans le stade C de l'exemple 1 en utilisant le produit obtenu dans le stade précédent.
**Spectre de masse : FAB**^{**+**} **: [M**^{**+**} **+ 1] : m/z = 524**

### Stade L : Acide 2-{[3-(benzylsulfanyl)-1(méthoxycarbonyl)propyl][(4-méthoxyphényl) sulfonyl]amino}acétique

Une solution de 0,8 mmol du composé obtenu dans le stade C dans 2 ml de dichlorométhane et de 3 ml d'acide trifluoroacétique est agitée 2 heures à température ambiante. Le milieu réactionnel est ensuite concentré sous pression réduite, puis le résidu est chromatographié sur gel de silice (dichlorométhane/méthanol : 95/5) permettant d'isoler le produit attendu.
**Spectre de masse : FAB**^{**+**} **: [M**^{**+**} **+ 1] : m/z = 468**

### Stade M : 2-{[2-(Benzhydrylamino)-2-oxoéthyl][(4-méthoxyphényl)sulfonyl]amino}-4 (benzylsulfanyl)butanoate de méthyle

A une solution de 1,5 mmol du composé obtenu dans le stade L, 1,6 mmol d'aminodiphénylméthane, dans 20 ml d'acétonitrile sont additionnés, à 0°C, 1,8 mmol de benzotriazol-1-yloxytris(diméthylamino)-phosphonium hexafluorophosphate puis 3 mmol de diisopropyléthylamine. Le milieu réactionnel est ensuite ramené lentement à température ambiante. Après 6 heures de réaction, l'acétonitrile est évaporé et le résidu est repris à l'acétate d'éthyle, puis lavé par une solution saturée en NaCl. Les phases organiques sont séchées sur sulfate de sodium, filtrées puis concentrées sous pression réduite. Une chromatographie sur gel de silice (dichlorométhane/méthanol : 20/1) permet d'isoler le produit attendu.
**Spectre de masse : FAB**^{**+**} **: [M**^{**+**} **+ 1] : m/z = 633**

### Stade N : Acide 2-{[2-(benzhydrylamino)-2-oxoéthyl][(4-méthoxyphényl) sulfonyl]amino}-4-(benzylsulfanyl)butanhydroxamique

On procède comme dans l'exemple 1 des stades D à F, en utilisant comme substrat le produit obtenu dans le stade M précédent. **Spectre de masse : FAB**^{**+**} **: [M**^{**+**} **+ 1] : m/z = 634**

### EXEMPLE 7 : Acide 2-{[2-(benzhydrylamino)-2-oxoéthyl][(4-biphényl) sulfonyl]amino}-4-(benzylsulfanyl)butanhydroxamique

Le produit est obtenu selon le procédé décrit dans l'exemple 6, en utilisant au stade B, comme réactif, le chlorure de 4-biphénylsulfonyle.

### EXEMPLE 8 : Acide 4-{[(1-méthyl-3-pipéridyl)méthyl]sulfanyl}-2-[isobutyl-(4-biphénylsulfonyl)amino]butanhydroxamique

Le produit est obtenu selon le procédé décrit dans l'exemple 1, des stades A à F, en utilisant comme réactif au stade B celui utilisé dans l'exemple 3, et au stade C, le 3-chlorométhyl-1-méthylpipéridine.

### EXEMPLE 9 : Acide 4-[(1-naphtylméthyl)sulfanyl]-2-{isobutyl][(4-méthoxyphényl) sulfonyl]amino}butanhydroxamique

Le produit est obtenu selon le procédé décrit dans l'exemple 1, des stades A à F, en utilisant comme réactif au stade C, le 1-(chlorométhyl)naphtalène.

### EXEMPLE 10 : Acide 4-{[(phénylsulfanyl)méthyl]sulfanyl]}-2-[isobutyl-(4-biphénylsulfonyl)amino]butanhydroxamique

Le produit est obtenu selon le procédé décrit dans l'exemple 1, des stades A à F, en utilisant comme réactif au stade B, celui utilisé dans l'exemple 3, et au stade C, le chlorure de méthylthiophényle.

### EXEMPLE 11 : Acide 4-[(3-pyridylméthyl)sulfanyl]-2-[isobutyl-(4-biphénylsulfonyl) aminol-butanhydroxamique

Le produit est obtenu selon le procédé décrit dans l'exemple 1, des stades A à F, en utilisant comme réactif au stade B, celui utilisé dans l'exemple 3, et au stade C, la 3-chlorométhylpyridine.

### EXEMPLE 12 : Acide 4-{[2-(tétrahydro-2H-pyranyl)méthyl]sulfanyl}-2-{isobutyl-[4-(méthoxyphényl)sulfonyl]amino}butanhydroxamique

Le produit est obtenu selon le procédé décrit dans l'exemple 1, des stades A à F, en utilisant comme réactif au stade C le 2-(chlorométhyl)-tétrahydro-2H-pyrane.

### EXEMPLE 13 : Acide 4-[(phénylsulfanyléthyl)sulfanyl]-2-{isobutyl-[4-(méthoxyphényl)sulfonyl]amino}butanhydroxamique

Le produit est obtenu selon le procédé décrit dans l'exemple 1, des stades A à F, en utilisant comme réactif au stade C, le chlorure d'éthylthiophényle.

### ETUDE PHARMACOLOGIQUE DES COMPOSES DE L'INVENTION

### EXEMPLE 14 : Inhibition enzymatique des métalloprotéases

Les tests enzymatiques de criblage des composés sont réalisés en solution sur tout ou partie des quatre enzymes humaines purifiées suivantes: collagénase interstitielle MMP-1, gélatinases MMP-2 et MMP-9, stromélysine-1 MMP-3. L'activité est révélée par une méthode fluorométrique adaptée à un format de plaque 96 puits.

### Activation des MMPs

Cette étape permet de convertir les proformes des métalloenzymes en forme activées capables de cliver les substrats utilisés. Les enzymes commerciales, aliquotées et conservées à -80°C, sont diluées dans un tampon 50 mM Tris, 200 mM NaCl, 5 mM CaCl₂, 0,1% Brij35, pH 7,7 à raison de 355 µg/ml (MMP-1), 444 µg/ml (MMP-2), 187 µg/ml (MMP-3) et 500 µg/ml (MMP-9) d'enzyme en présence de 2 mM d'APMA (4-aminophenylmercuric acetate) à 37°C pendant 30 minutes (MMP-2 et MMP-9) ou 1 heure (MMP-1 et MMP-3).

### Test fluorogénique

Le principe repose sur l'apparition d'une fluorescence après clivage d'un peptide pseudosubstrat en présence de l'enzyme activée. Le peptide Dnp-Pro-β-cyclohexyl-Ala-Gly-Cys(Me)-His-Ala-Lys(N-Me-Abz)-NH₂ (Bachem, Suisse) est clivé entre la glycine et la cystéine (Anal. Biochem. 1993, 212, 58-64) par les enzymes activées MMP-1, MMP-2 et MMP-9. Le peptide (7-méthoxycoumarine-4-yl)-Arg-Pro-Lys-Pro-Tyr-Ala-Nva-Trp-Met-Lys(Dnp)-NH₂ (Bachem) est clivé entre Ala et Nva (Anal. Biochem. 1993, 212, 58-64) par l'enzyme activée MMP-3 (Biochemistry 1992, 31, 12618-12623). Les tests sont réalisés dans le tampon 50 mM Tris, 200 mM NaCI, 5 mM CaCl₂, 0.1% Brij35, pH 7.7 contenant les enzymes purifiées diluées (aux concentrations finales: 1,25, 2, 1,25 et 1 µg/ml pour les enzymes MMP-1, MMP-2, MMP-3 et MMP-9, respectivement). Après préincubation des enzymes avec ou sans les produits testés (minimum de cinq doses par dilutions de dix en dix), les réactions de clivage sont initiées en ajoutant 20 µM (concentration finale) du peptide pseudosubstrat approprié dans un volume final total de 100 µl (format plaques de 96 puits). Après six heures d'incubation à 37°C en atmosphère humide, les plaques contenant les échantillons sont lues dans un cytofluorimètre (Cytofluor 2350, Millipore PerSeptive Systems, France) équipé d'une combinaison de filtres d'excitation et d'émission de 340 et 440 nm, respectivement. Chaque condition est réalisée en triplicat. La concentration inhibant 50% de la réaction (IC₅₀) est alors déterminée à partir des courbes représentant l'intensité de fluorescence des produits de clivage en fonction des doses testées. Chaque expérience est réalisée au moins deux fois.

Lors de ce test, les composés de l'invention ont présentées des IC₅₀ comprises entre 100 et 200 nM pour l'enzyme MMP-1, entre 0,2 et 50 nM pour les enzymes MMP-2, MMP-3 et MMP-9.

### EXEMPLE 15 : Composition pharmaceutique : comprimés

Formule de préparation pour 1000 comprimés dosés à 20 mg

| | |
|---|---|
| Composé de l'exemple 2 | 20g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 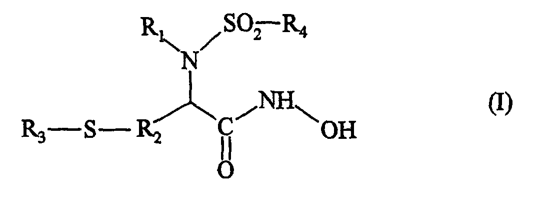 dans laquelle :
R₁ représente
- un groupement alkyle C₁-C₆ linéaire ou ramifié (éventuellement substitué par un ou plusieurs groupements, identiques ou différents indépendamment l'un de l'autre, choisis parmi hydroxy, halogène, alkoxy C₁-C₆ linéaire ou ramifié, mercapto, alkylthio C₁-C₆ linéaire ou ramifié, aryle, acyle C₁-C₆ linéaire ou ramifié, ou amino lui-même éventuellement substitué par un ou deux groupements, identiques ou différents, alkyle C₁-C₆ linéaire ou ramifié, cycloalkyle ou aryle),
- un groupement acyle C₁-C₆ linéaire ou ramifié,
- un groupement cycloalkyle,
- un groupement aryle,
- un hétérocycle,
- un groupement aminocarbonylalkyle C₁-C₄, la partie amino étant éventuellement substituée par un groupement alkyle C₁-C₆ linéaire ou ramifié, ledit groupement alkyle étant éventuellement substitué par un ou plusieurs groupements, identiques ou différents, choisis parmi aryle, arylalkyle C₁-C₆ linéaire ou ramifié, cycloalkyle ou alkylaminocarbonyle C₁-C₆ linéaire ou ramifié,
R₂ représente un groupement alkylène C₁-C₄ linéaire ou ramifié,
R₃ représente un groupement X ou Y avec :
• X représente un groupement alkyle C₁-C₆ linéaire ou ramifié, acyle C₁-C₆ linéaire ou ramifié, alkoxycarbonyle C₁-C₆ linéaire ou ramifié, aminoalkyle C₁-C₆ linéaire ou ramifié (la partie amino étant elle-même éventuellement substituée par un ou deux groupements, identiques ou différents, alkyle C₁-C₆ linéaire ou ramifié), hydroxyalkyle C₁-C₆ linéaire ou ramifié, carboxyalkyle C₁-C₆ linéaire ou ramifié, aminocarbonylalkyle C₁-C₆ linéaire ou ramifié, mercaptoalkyle C₁-C₆ linéaire ou ramifié, cycloalkyle, aryle ou un hétérocycle,
• Y représente un groupement de formule T-U-V- (la partie V étant reliée à l'atome de soufre) dans laquelle :
- T représente un groupement aryle ou un hétérocycle,
- U représente une liaison simple, un atome de soufre, d'oxygène, un groupement NH ou C=O, ou un groupement de formules -R₈O-, -R₈S-, -R₈NH-, -R₈OR₉, -R₈SR₉-, -R₈-NH-R₉-, -R₈-CO-R₉-, -R₉-, dans lesquelles R₈ représente un groupement alkylène C₁-C₆ linéaire ou ramifié, et R₉ représente un groupement arylène ou hétéroaryléne, étant entendu que dans cesdits groupements, R₈ est relié à la partie T du groupement Y et R₉ ou l'hétéroatome est relié à la partie V du groupement Y,
- V représente un groupement alkylène C₁-C₆ linéaire ou ramifié,
R₄ représente :
- soit, quand R₃ représente un groupement Y, un groupement alkyle C₁-C₆ linéaire ou ramifié, cycloalkyle, aryle, arylalkyle C₁-C₆ linéaire ou ramifié, cycloalkylalkyle C₁-C₆ linéaire ou ramifié, alkyle C₁-C₆ linéaire ou ramifié substitué par un hétérocycle, ou un hétérocycle,
- soit, quand R₃ représente un groupement X ou Y, un groupement biaryle, arylhétéroaryle, ou hétéroarylaryle,
leurs isomères ainsi que leurs sels d'addition à un acide ou à une base pharmaceutiquement acceptable.

2. Composés de formule (I) selon la revendication 1 **caractérisé en ce que** R₃ représente un groupement X tel que défini précédemment, et R₄ représente un groupement biaryle, arylhétéroaryle ou hétéroarylaryle, leurs isomères ainsi que leurs sels d'addition à un acide ou à une base pharmaceutiquement acceptable.

3. Composés de formule (I) selon la revendication 1 **caractérisé en ce que** R₃ représente un groupement Y tel que défini précédemment, et R₄ représente un groupement alkyle C₁-C₆ linéaire ou ramifié, cycloalkyle, aryle, arylalkyle C₁-C₆ linéaire ou ramifié, cycloalkylalkyle C₁-C₆ linéaire ou ramifié, hétérocycloalkylène C₁-C₆ linéaire ou ramifié ou un hétérocycle, leurs isomères ainsi que leurs sels d'addition à un acide ou à une base pharmaceutiquement acceptable.

4. Composés de formule (I) selon la revendication 1 **caractérisé en ce que** R₃ représente un groupement Y, et R₄ représente un groupement biaryle, arylhétéroaryle, ou hétéroarylaryle, leurs isomères ainsi que leurs sels d'addition à un acide ou à une base pharmaceutiquement acceptable.

5. Composés de formule (I) selon la revendication 1 **caractérisés en ce que** R₁ représente un groupement alkyle C₁-C₆ linéaire ou ramifié, leurs isomères ainsi que leurs sels d'addition à un acide ou à une base pharmaceutiquement acceptable.

6. Composés de formule (I) selon l'une quelconque des revendications 1 et 5 **caractérisés en ce que** R₁ représente un groupement isobutyle, leurs isomères ainsi que leurs sels d'addition à un acide ou à une base pharmaceutiquement acceptable.

7. Composé de formule (I) selon la revendication 1 qui est l'acide 4-(benzylsulfanyl)-2-{isobutyl[(4-méthoxyphényl)sulfonyl]amino}butanhydroxamique.

8. Composé de formule (I) selon la revendication 1 qui est l'acide 4-{[4-(phényl)benzyle]sulfanyl}-2-{isobutyl-[(4-méthoxyphényl)sulfonyl]amino}butanhydroxamique.

9. Composé de formule (I) selon la revendication 1 qui est l'acide 4-(benzylsulfanyle)-2-{isobutyl-[(4-biphényl)sulfonyl]amino}butanhydroxamique.

10. Composé de formule (I) selon la revendication 1 qui est l'acide 4-{[4-(benzyloxy)benzyl]sulfanyl}-2-{isobutyl-[(4-méthoxyphényl)sulfonyl]amino}butanhydroxamique.

11. Composé de formule (I) selon la revendication 1 qui est l'acide 2-{isobutyl-[(4-biphényl)sulfonyl]amino}-4-(méthylsulfanyl)butanhydroxamique.

12. Composé de formule (I) selon la revendication 1 qui est l'acide 2-{[2-(benzhydrylamino)-2-oxoéthyl]-[(4-méthoxyphényl)sulfonyl]amino}-4-(benzylsulfanyl) butanhydroxamique.

13. Procédé de préparation des composés de formule (I) selon la revendication 1, **caractérisé en ce que** l'on utilise comme produit de départ, un composé de formule (II) : 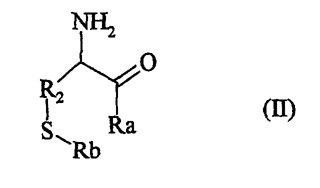 dans laquelle R₂ est tel que défini dans la formule (I) et, soit Ra et Rb forment une liaison simple reliant le groupement carbonyle à l'atome de soufre, soit Ra représente un groupement alkoxy C₁-C₆ linéaire ou ramifié, et Rb représente un groupement alkyle C₁-C₆ linéaire ou ramifié,
composés de formule (II), dont on substitue, selon des conditions classiques de synthèse organique, la fonction amine primaire, par un composé de formule (II') :
R₁ - Z (II')
dans laquelle R₁ est tel que défini dans la formule (I) et Z représente un groupement libérable usuel de la chimie organique,
pour conduire aux composés de formule (III), 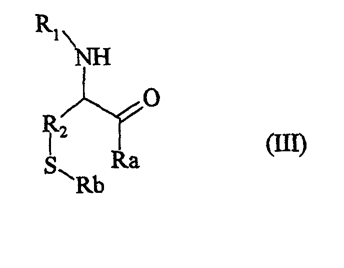 dans laquelle R₁ et R₂ ont la même signification que dans la formule (I) et, Ra et Rb sont tels que définis précédemment,
composés de formule (III), que l'on traite en condition basique, avec un composé de formule (IV) :
R₄ - SO₂ - Cl (IV)
dans laquelle R₄ est tel que défini dans la formule (I),
pour conduire aux composés de formule (V), 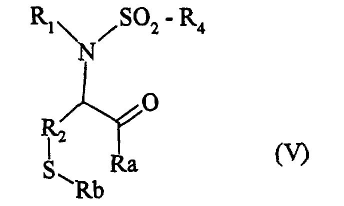 dans laquelle R₁, R₂, R₄, Ra et Rb sont tels que définis précédemment,
composés de formule (V) que l'on traite, dans le cas où Ra et Rb forment une liaison simple, en présence de méthanol et de sodium, par un composé de formule (VI) :
R₃ - Hal (VI)
dans laquelle R₃ est tel que défini dans la formule (I) et Hal représente un atome d'halogène, pour conduire aux composés de formule (VII) : 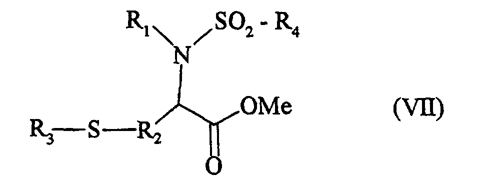 dans laquelle R₁, R₂, R₃ et R₄ sont tels que définis dans la formule (I), l'ensemble des composés de formule (VII) et (V) formant les composés de formule (IX) : 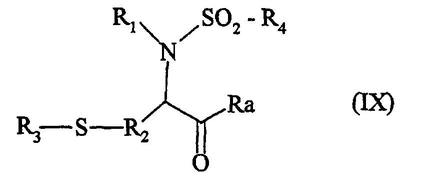 dans laquelle R₁, R₂, R₃, R₄ et Ra sont tels que définis précédemment,
composés de formule (IX) dont on hydrolyse la fonction ester, selon des conditions classiques, pour conduire aux composés de formule (X) : 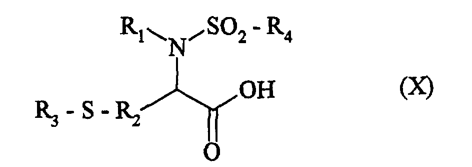 dans laquelle R₁, R₂, R₃ et R₄ sont tels que définis précédemment,
composés de formule (X) que l'on traite soit directement par de l'hydrochlorure d'hydroxylamine, soit par une hydroxylamine-O-substituée que l'on déprotège par la suite selon des conditions opératoires classiques, pour conduire aux composés de formule (I) tels que définis précédemment,
composé de formule (I) que l'on purifie, le cas échéant, selon une technique classique de purification, dont on sépare éventuellement les isomères selon une technique classique de séparation et que l'on transforme, si on le souhaite, en ses sels d'addition à un acide ou à une base pharmaceutiquement acceptable.

14. Compositions pharmaceutiques contenant comme principe actif au moins un composé de formule (I) selon l'une quelconque des revendications 1 à 12, seul ou en combinaison avec un ou plusieurs excipients ou véhicules inertes, non toxiques, pharmaceutiquement acceptables.

15. Compositions pharmaceutiques selon la revendication 14 contenant au moins un principe actif selon l'une quelconque des revendications 1 à 12, utiles comme inhibiteurs de métalloprotéases.

## Claims

1. Compounds of formula (I) : 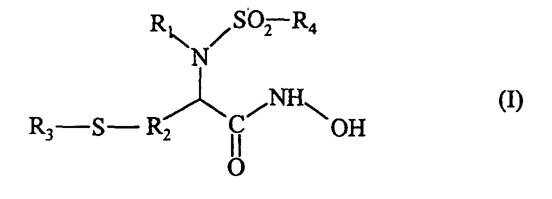 wherein :
R₁ represents :
- a linear or branched C₁-C₆alkyl group (optionally substituted by one or more identical or different groups each selected independently of the other(s) from hydroxy, halogen, linear or branched C₁-C₆alkoxy, mercapto, linear or branched C₁-C₆alkylthio, aryl, linear or branched C₁-C₆acyl, and amino, which is itself optionally substituted by one or two identical or different linear or branched C₁-C₆alkyl, cycloalkyl or aryl groups),
- a linear or branched C₁-C₆acyl group,
- a cycloalkyl group,
- an aryl group,
- a heterocycle,
- an aminocarbonyl-C₁-C₄alkyl group, the amino moiety being optionally substituted by a linear or branched C₁-C₆alkyl group, the said alkyl group being optionally substituted by one or more identical or different groups selected from aryl, aryl-C₁-C₆alkyl in which the alkyl moiety may be linear or branched, cycloalkyl and linear or branched C₁-C₆alkylaminocarbonyl,
R₂ represents a linear or branched C₁-C₄alkylene group,
R₃ represents a group X or Y:
• X representing a linear or branched C₁-C₆alkyl group, a linear or branched C₁-C₆acyl group, a linear or branched C₁-C₆alkoxycarbonyl group, a linear or branched amino-C₁-C₆alkyl group (the amino moiety itself being optionally substituted by one or two identical or different linear or branched C₁-C₆alkyl groups), a linear or branched hydroxy-C₁-C₆alkyl group, a linear or branched carboxy-C₁-C₆alkyl group, a linear or branched aminocarbonyl-C₁-C₆alkyl group, a linear or branched mercapto-C₁-C₆alkyl group, a cycloalkyl group, an aryl group or a heterocycle, and
• Y representing a group of formula T-U-V- (the moiety V being bonded to the sulphur atom), in which :
- T represents an aryl group or a heterocycle,
- U represents a single bond, a sulphur atom, an oxygen atom, an NH or C=O group, or a group of formula -R₈O-, -R₈S-, -R₈NH-, -R₈OR₉- -R₈SR₉-, -R₈-NH-R₉-, -R₈-CO-R₉- or -R₉- in which R₈ represents a linear or branched C₁-C₆alkylene group and R₉ represents an arylene or heteroarylene group, with the proviso that in those groups R₈ is bonded to the T moiety of the group Y and R₉ or the hetero atom is bonded to the V moiety of the group Y,
- V represents a linear or branched C₁-C₆alkylene group,
R₄ represents :
- either, when R₃ represents a group Y: a linear or branched C₁-C₆alkyl group, a cycloalkyl group, an aryl group, an aryl-C₁-C₆alkyl group in which the alkyl moiety may be linear or branched, a cycloalkyl-C₁-C₆alkyl group in which the alkyl moiety may be linear or branched, a linear or branched C₁-C₆alkyl group substituted by a heterocycle, or a heterocycle,
- or, when R₃ represents a group X or Y: a biaryl group, an arylheteroaryl group, or a heteroarylaryl group,
isomers thereof, and also addition salts thereof with a pharmaceutically acceptable acid or base.

2. Compounds of formula (I) according to claim 1, **characterised in that** R₃ represents a group X as defined hereinbefore, and R₄ represents a biaryl, arylheteroaryl or heteroarylaryl group, isomers thereof, and also addition salts thereof with a pharmaceutically acceptable acid or base.

3. Compounds of formula (I) according to claim 1, **characterised in that** R₃ represents a group Y as defined hereinbefore and R₄ represents a linear or branched C₁-C₆alkyl group, a cycloalkyl group, an aryl group, an aryl-C₁-C₆alkyl group in which the alkyl moiety may be linear or branched, a cycloalkyl-C₁-C₆alkyl group in which the alkyl moiety may be linear or branched, a linear or branched C₁-C₆heterocycloalkylene group, or a heterocycle, isomers thereof, and also addition salts thereof with a pharmaceutically acceptable acid or base.

4. Compounds of formula (I) according to claim 1, **characterised in that** R₃ represents a group Y and R₄ represents a biaryl, arylheteroaryl or heteroarylaryl group, isomers thereof, and also addition salts thereof with a pharmaceutically acceptable acid or base.

5. Compounds of formula (I) according to claim 1, **characterised in that** R₁ represents a linear or branched C₁-C₆alkyl group, isomers thereof, and also addition salts thereof with a pharmaceutically acceptable acid or base.

6. Compounds of formula (I) according to any one of claims 1 to 5, **characterised in that** R₁ represents an isobutyl group, isomers thereof, and also addition salts thereof with a pharmaceutically acceptable acid or base.

7. Compound of formula (I) according to claim 1, which is 4-(benzylsulphanyl)-2-{isobutyl[(4-methoxyphenyl)sulphonyl]amino}butanehydroxamic acid.

8. Compound of formula (I) according to claim 1, which is 4-{[4-(phenyl)-benzyl]sulphanyl}-2-{isobutyl-[(4-methoxyphenyl)sulphonyl]amino}butanehydroxamic acid.

9. Compound of formula (I) according to claim 1, which is 4-(benzylsulphanyl)-2-{isobutyl-[(4-biphenyl)sulphonyl]amino}butanehydroxamic acid.

10. Compound of formula (I) according to claim 1, which is 4-{[4-(benzyloxy)benzyl]-sulphanyl}-2-{isobutyl-[(4-methoxyphenyl}sulphonyl]amino}butanehydroxamic acid.

11. Compound of formula (I) according to claim 1, which is 2-{isobutyl-[(4-biphenyl)-sulphonyl]amino}-4-(methylsulphanyl)butanehydroxamic acid.

12. Compound of formula (I) according to claim 1, which is 2-{[2-(benzhydrylamino)-2-oxoethyl]-[(4-methoxyphenyl)sulphonyl]amino}-4-(benzylsulphanyl)butanehydroxamic acid.

13. Process for the preparation of compounds of formula (I) according to claim 1, **characterised in that** there is used as starting material a compound of formula (II) : 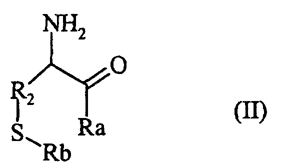 wherein R₂ is as defined for formula (I) and either Ra and Rb form a single bond connecting the carbonyl group to the sulphur atom, or Ra represents a linear or branched C₁-C₆alkoxy group and Rb represents a linear or branched C₁-C₆alkyl group,
the primary amine function of which compound of formula (II) is substituted, according to conventional conditions of organic synthesis, by a compound of formula (II') :
R₁ - Z (II')
wherein R₁ is as defined for formula (I) and Z represents a leaving group customary in organic chemistry,
to yield a compound of formula (III), 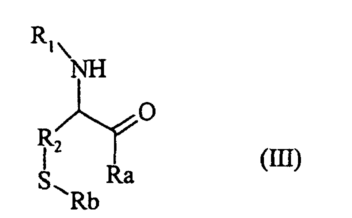 wherein R₁ and R₂ are as defined for formula (I) and Ra and Rb are as defined hereinbefore,
which compound of formula (III) is treated under basic conditions with a compound of formula (IV) :
R₄ - SO₂ - Cl (IV)
wherein R₄ is as defined for formula (I),
to yield a compound of formula (V), 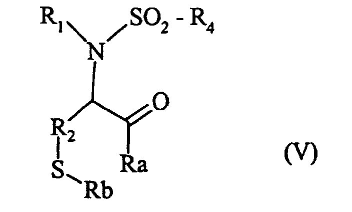 wherein R₁, R₂, R₄, Ra and Rb are as defined hereinbefore,
which compound of formula (V), when Ra and Rb form a single bond, is treated in the presence of methanol and sodium with a compound of formula (VI) :
R₃ - Hal (VI)
wherein R₃ is as defined for formula (I) and Hal represents a halogen atom,
to yield a compound of formula (VII) : 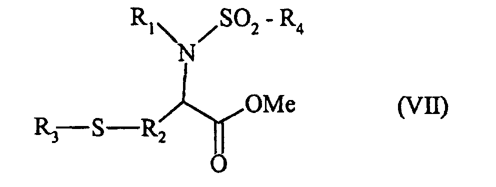 wherein R₁, R₂, R₃ and R₄ are as defined for formula (I), the totality of the compounds of formulae (VII) and (V) constituting the compounds of formula (IX) : 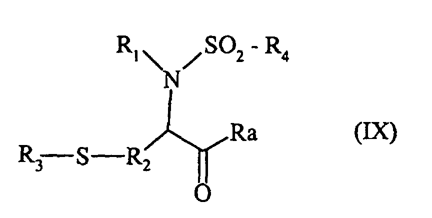 wherein R₁, R₂, R₃, R₄ and Ra are as defined hereinbefore,
the ester function of which compound of formula (IX) is hydrolysed according to conventional conditions to yield a compound of formula (X) : 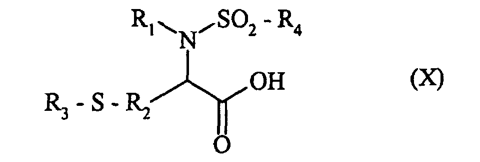 wherein R₁, R₂, R₃ and R₄ are as defined hereinbefore,
which compound of formula (X) is treated either directly with hydroxylamine hydrochloride, or with an O-substituted hydroxylamine which is deprotected subsequently according to conventional operating conditions, to a yield compound of formula (I) as defined hereinbefore,
which compound of formula (I) is, if necessary, purified according to a conventional purification technique, is optionally separated into its isomers according to a conventional separation technique and is, if desired, converted into an addition salt with a pharmaceutically acceptable acid or base.

14. Pharmaceutical compositions comprising as active ingredient at least one compound of formula (I) according to any one of claims 1 to 12, alone or in combination with one or more pharmaceutically acceptable, inert, non-toxic excipients or carriers.

15. Pharmaceutical compositions according to claim 14 comprising at least one active ingredient according to any one of claims 1 to 12 for use as metalloprotease inhibitors.

## Patentansprüche

1. Verbindungen der Formel (I): 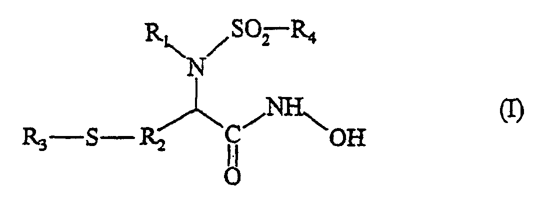 in der:
R₁:
- eine geradkettige oder verzweigte C₁-C₆-Alkylgruppe (gegebenenfalls substituiert durch eine oder mehrere gleichartige oder verschiedene Gruppen, die unabhängig voneinander ausgewählt sind aus Hydroxy, Halogen, geradkettigem oder verzweigtem C₁-C₆-Alkoxy, Mercapto, geradkettigem oder verzweigtem C₁-C₆-Alkylthio, Aryl, geradkettigem oder verzweigtem C₁-C₆-Acyl oder Amino, das gegebenenfalls seinerseits durch eine oder zwei gleichartige oder verschiedene, geradkettige oder verzweigte C₁-C₆-Alkylgruppen, Cycloalkylgruppen oder Arylgruppen substituiert ist),
- eine geradkettige oder verzweigte C₁-C₆-Acylgruppe,
- eine Cycloalkylgruppe,
- eine Arylgruppe,
- einen Heterocyclus,
- eine Aminocarbonyl-C₁-C₄-alkylgruppe, deren Aminorest gegebenenfalls durch eine geradkettige oder verzweigte C₁-C₆-Alkylgruppe substituiert ist, welche Alkylgruppe gegebenenfalls substituiert ist durch eine oder mehrere gleichartige oder verschiedene Gruppen ausgewählt aus Aryl, geradkettigem oder verzweigtem Aryl-C₁-C₆-alkyl, Cycloalkyl oder geradkettigem oder verzweigtem C₁-C₆-Alkylaminocarbonyl, bedeutet,
R₂ eine geradkettige oder verzweigte C₁-C₄-Alkylengruppe darstellt,
R₃ eine Gruppe X oder Y darstellt, worin:
. X eine geradkettige oder verzweigte C₁-C₆-Alkylgruppe, geradkettige oder verzweigte C₁-C₆-Acylgruppe, geradkettige oder verzweigte C₁-C₆-Alkoxycarbonylgruppe, geradkettige oder verzweigte Amino-C₁-C₆-alkylgruppe (wobei der Aminorest seinerseits gegebenenfalls durch eine oder zwei gleichartige oder verschiedene geradkettige oder verzweigte C₁-C₆-Alkylgruppen substituiert ist), geradkettige oder verzweigte C₁-C₆-Hydroxyalkylgruppe, geradkettige oder verzweigte Carboxy-C₁-C₆-alkylgruppe, geradkettige oder verzweigte Aminocarbonyl-C₁-C₆-alkylgruppe, geradkettige oder verzweigte Mercapto-C₁-C₆-alkylgruppe, Cycloalkylgruppe, Arylgruppe oder einen Heterocyclus darstellt,
. Y eine Gruppe der Formel T-U-V- bedeutet (worin der Rest V an ein Schwefelatom gebunden ist), in der:
- T eine Arylgruppe oder einen Heterocyclus darstellt,
- U eine Einfachbindung, ein Schwefelatom, ein Sauerstoffatom, eine Gruppe NH oder C=O oder eine Gruppe der Formeln -R₈O-, -R₈S-, -R₈NH-, -R₈OR₉, -R₈SR₉-, -R₈-NH-R₉-, -R₈-CO-R₉-, -R₉-, worin R₈ eine geradkettige oder verzweigte C₁-C₆-Alkylengruppe und R₉ eine Arylen- oder Heteroarylengruppe darstellen, mit der Maßgabe bedeutet, daß bei diesen Gruppen R₈ an den Rest T der Gruppe Y und R₉ oder das Heteroatom an den Rest V der Gruppe Y gebunden sind,
- V eine geradkettige oder verzweigte C₁-C₆-Alkylengruppe bedeutet,
R₄:
- entweder, wenn R₃ eine Gruppe Y darstellt, eine geradkettige oder verzweigte C₁-C₆-Alkylgruppe, Cycloalkylgruppe, Arylgruppe, geradkettige oder verzweigte Aryl-C₁-C₆-alkylgruppe, geradkettige oder verzweigte Cycloalkyl-C₁-C₆-alkylgruppe, geradkettige oder verzweigte, durch einen Heterocyclus substituierte C₁-C₆-Alklgruppe oder einen Heterocyclus bedeutet,
- oder, wenn R₃ eine Gruppe X oder Y darstellt, eine Biaryl-, Arylheteroaryloder Heteroarylarylgruppe darstellt,
deren Isomere sowie deren Additionssalze mit einer pharmazeutisch annehmbaren Säure oder Base.

2. Verbindungen der Formel (I) nach Anspruch 1, **dadurch gekennzeichnet, daß** R₃ eine Gruppe X, wie sie oben definiert worden ist, und R₄ eine Biaryl-, Arylheteroaryl- oder Heteroarylarylgruppe bedeuten, deren Isomere sowie deren Additionssalze mit einer pharmazeutisch annehmbaren Säure oder Base.

3. Verbindungen der Formel (I) nach Anspruch 1, **dadurch gekennzeichnet, daß** R₃ eine Gruppe Y, wie sie oben definiert worden ist, und R₄ eine geradkettige oder verzweigte C₁-C₆-Alkylgruppe, Cycloalkylgruppe, Arylgruppe, geradkettige oder verzweigte Aryl-C₁-C₆-alkylgruppe, geradkettige oder verzweigte Cycloalkyl-C₁-C₆-alkylgruppe, geradkettige oder verzweigte Heterocyclo-C₁-C₆-alkylengruppe oder einen Heterocyclus bedeuten, deren Isomere sowie deren Additionssalze mit einer pharmazeutisch annehmbaren Säure oder Base.

4. Verbindungen der Formel (I) nach Anspruch 1, **dadurch gekennzeichnet, daß** R₃ eine Gruppe Y und R₄ eine Biaryl-, Arylheteroaryl- oder Heteroarylarylgruppe bedeuten, deren Isomere sowie deren Additionssalze mit einer pharmazeutisch annehmbaren Säure oder Base.

5. Verbindungen der Formel (I) nach Anspruch 1, **dadurch gekennzeichnet, daß** R₁ eine geradkettige oder verzweigte C₁-C₆-Alkylgruppe bedeutet, deren Isomere sowie deren Additionssalze mit einer pharmazeutisch annehmbaren Säure oder Base.

6. Verbindungen der Formel (I) nach irgendeinem der Ansprüche 1 und 5, **dadurch gekennzeichnet, daß** R₁ eine Isobutylgruppe bedeutet, deren Isomere sowie deren Additionssalze mit einer pharmazeutisch annehmbaren Säure oder Base.

7. Verbindung der Formel (I) nach Anspruch 1, nämlich 4-(Benzylsulfanyl)-2-{isobutyl-[(4-methoxyphenyl)-sulfonyl]-amino}-butanhydroxamsäure.

8. Verbindung der Formel (I) nach Anspruch 1, nämlich 4-{[4-(Phenyl)-benzyl]-sulfanyl}-2-{isobutyl-[(4-methoxyphenyl)-sulfonyl]-amino}-butanhydroxamsäure.

9. Verbindung der Formel (I) nach Anspruch 1, nämlich 4-(Benzylsulfanyl)-2-{isobutyl-[(4-biphenyl)-sulfonyl]-amino}-butanhydroxamsäure.

10. Verbindung der Formel (I) nach Anspruch 1, nämlich 4-{[4-(Benzyloxy)-benzyl]-sulfanyl}-2-{isobutyl-[(4-methoxyphenyl)-sulfonyl]-amino}-butanhydroxamsäure.

11. Verbindung der Formel (I) nach Anspruch 1, nämlich 2-{Isobutyl-[(4-biphenyl)-sulfonyl]-amino}-4-(methylsulfanyl)-butanhydroxamsäure.

12. Verbindung der Formel (I) nach Anspruch 1, nämlich 2-{[2-(Benzhydrylamino)-2-oxoethyl]-[(4-methoxyphenyl)-sulfonyl]-amino}-4-(benzylsulfanyl)-butanhydroxamsäure.

13. Verfahren zur Herstellung der Verbindungen der Formel (I) nach Anspruch 1. **dadurch gekennzeichnet, daß** man als Ausgangsprodukt eine Verbindung der Formel (II) verwendet: 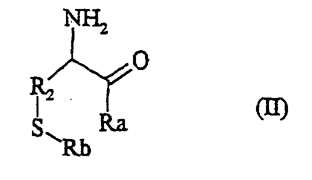 in der R₂ die bezüglich der Formel (I) angegebenen Bedeutungen besitzt, und entweder Ra und Rb eine Einfachbindung bilden, welche die Carbonylgruppe mit dem Schwefelatom verbindet, oder Ra eine geradkettige oder verzweigte C₁-C₆-Alkoxygruppe und Rb eine geradkettige oder verzweigte C₁-C₆-Alkylgruppe bedeuten,
bei welchen Verbindungen der Formel (II) man die primäre Aminfunktion gemäß klassischen Methoden der organischen Chemie mit einer Verbindung der Formel (II') substituiert:
R₁ - Z (II')
in der R₁ die bezüglich der Formel (I) angegebenen Bedeutungen besitzt und Z eine übliche freisetzbare Gruppe der organischen Chemie darstellt,
zur Bildung der Verbindungen der Formel (III) 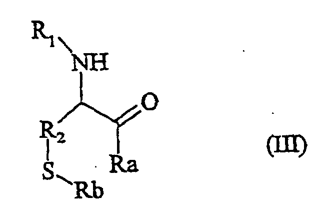 in der R₁ und R₂ die bezüglich der Formel (I) angegebenen Bedeutungen besitzen und Ra und Rb die oben angegebenen Bedeutungen aufweisen,
welche Verbindungen der Formel (III) man unter basischen Bedingungen mit einer Verbindung der Formel (IV) behandelt:
R₄ - SO₂ - Cl (IV)
in der R₄ die bezüglich der Formel (I) angegebenen Bedeutungen besitzt,
zur Bildung der Verbindungen der Formel (V) 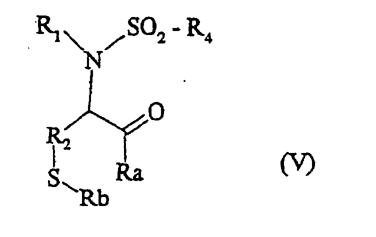 in der R₁, R₂, R₄, Ra und Rb die oben angegebenen Bedeutungen besitzen,
welche Verbindungen der Formel (V) man dann, wenn Ra und Rb eine Einfachbindung bilden, in Gegenwart von Methanol und Natrium mit einer Verbindung der Formel (VI) behandelt:
R₃ - Hal (VI)
in der R₃ die bezüglich der Formel (I) angegebenen Bedeutungen besitzen und Hal ein Halogenatom darstellt,
zur Bildung der Verbindungen der Formel (VII): 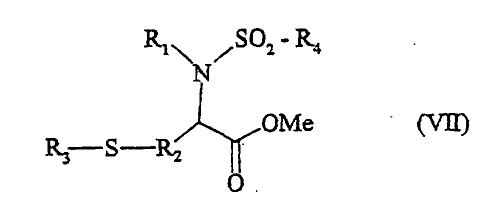 in der R₁, R₂, R₃ und R₄ die bezüglich der Formel (I) angegebenen Bedeutungen besitzen, wobei die Gesamtheit der Verbindungen der Formeln (VII) und (V) die Verbindungen der Formel (IX) bilden: 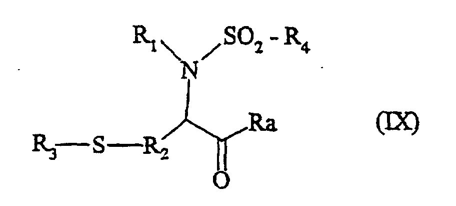 in der R₁, R₂, R₃, R₄ und Ra die oben angegebenen Bedeutungen besitzen,
bei welchen Verbindungen der Formel (IX) man die Esterfunktion unter klassischen Bedingungen hydrolysiert zur Bildung der Verbindungen der Formel (X): 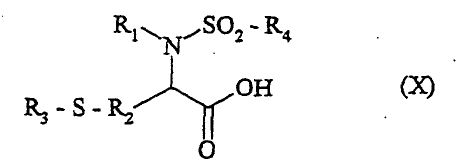 in der R₁, R₂, R₃ und R₄ die oben angegebenen Bedeutungen besitzen,
welche Verbindungen der Formel (X) man entweder direkt mit Hydroxylamin-Hydrochlorid oder mit O-substituiertem Hydroxylamin behandelt, wovon man anschließend mit Hilfe klassischer Methoden die Schutzgruppen abspaltet zur Bildung der Verbindungen der Formel (I), wie sie oben definiert worden sind,
welche Verbindung der Formel (I) man gegebenenfalls mit Hilfe klassischer Reinigungsmethoden reinigt, die man gegebenenfalls mit Hilfe einer klassischen Trennmethode in ihre Isomeren auftrennt und die man gewünschtenfalls in ihre Additionssalze mit einer pharmazeutisch annehmbaren Säure oder Base umwandelt.

14. Pharmazeutische Zubereitungen enthaltend als Wirkstoff mindestens eine Verbindung der Formel (I) nach irgendeinem der Ansprüche 1 bis 12 allein oder in Kombination mit einem oder mehreren inerten, nichttoxischen, pharmazeutisch annehmbaren Trägermaterialien oder Bindemitteln.

15. Pharmazeutische Zubereitungen nach Anspruch 14, enthaltend mindestens einen Wirkstoff nach irgendeinem der Ansprüche 1 bis 12 nützlich als Inhibitoren von Metallproteasen.
